# EUROPEAN PATENT APPLICATION

(11) **EP 2 014 180 A1**
(43) Date of publication of application: **14.01.2009**
(21) Application number: 08009476.6
(22) Date of filing: 23.05.2008
(51) Int. Cl.: A23L 1/30, A23L 1/302, A23L 1/308, A61K 31/202, A61K 31/716, A21D 13/00, A23L 2/52, A61P 9/10

(54) **Composition useful for the reduction of the cardivascular risk and foods that contain it**

(30) Priority: 08.07.2007 EP 07425361
(71) Applicant: Barilla G. e R. Fratelli S.p.A., 43100 Parma (IT)
(72) Inventor: Melegari, Camilla, 43100 Parma (IT); De Albertis, Pietro, Loc. Monticelli Terme 43022 Monteschiagugolo (IT)
(74) Representative: Ferreccio, Rinaldo

(57) **Abstract**

A composition is described suitable for addition to drinks and foods, the continued intake of which contributes to a decrease in the risk of cardiovascular disease, comprising the following active ingredients: beta glucans, at least one omega-3 fatty acid chosen among eicosapentaenoic acid (EPA) and docosahexaenoic acid (DHA) or triglycerides that contain it, vitamin E and folates; a bakery product and a drink are also described which are nutritionally balanced and which contain the aforementioned composition.

## Description

### Field of application

The present invention relates in general to the food and dietary industry sector.

In particular, the invention relates to a food composition suitable for being used in the production of functional foods capable of promoting the health of human beings, with particular reference to the cardiovascular system. The invention moreover relates to foods containing the aforementioned composition.

### Prior art

It has been known for several decades now that there are various risk factors which predispose the cardiovascular system to sometimes very serious and even lethal events, which include heart attacks and strokes. Some of these risk factors cannot be modified (diabetes, for example), while others can be modified and corrected. The risk factors of the latter category include arterial hypertension, hyperlipidaemia, hyperhomocysteinaemia, obesity, sedentariness, smoking and stress.

Regarding several of the abovementioned risk factors, correction can be achieved with the aid of drugs (such as antihypertensive and cholesterol-lowering drugs) but also, and preferably when possible, by a change and a supplementation of the diet. This is the case, for example, in hyperlipidaemia and hyperhomocysteinaemia.

A number of functional food products have therefore been developed containing ingredients capable of exerting a preventing action towards the cardiovascular system. For example, patent EP 1 411 960 describes a rice bran-based food product, enriched with the addition of various components: vitamins B₁, C, B₆, B₁₂ and E, folic acid and omega-3 fatty acids, in specific quantities and relative proportions. In the patent in question, it is briefly mentioned that other components, including beta-glucans, may be added, but no specific example is provided, nor is there any mention of any special effect obtained by means of such addition.

Another composition containing functional ingredients useful for the prevention of the risk of cardiovascular disease is described in US application 2006/0116334; such composition comprises a folate, one or more vitamins chosen among vitamins B₂, B₆ and B₁₂ or their metabolites or derivatives, and phytosterols. The composition can moreover comprise vitamin E, omega-3 fatty acids and policosanols.

In application WO 02/43662, a dietary composition is described for the reduction of the risk of cardiovascular disease, comprising conjugated linoleic acid, DHA, vitamins E, C, B₆, B₁₂, folic acid and calcium.

Patent US 6 210 686 describes a food supplement comprising fibre (for example beta-glucans), folic acid and vitamin E for the improvement of cardiovascular health.

In a first aspect thereof, the present invention is directed to making available a composition suitable for addition to drinks and foods, the continued intake of which contributes towards a decrease in the risk of cardiovascular disease.

In a second aspect, the present invention relates to foods and drinks containing the aforementioned composition and exhibiting a correct and balanced nutritional profile.

### Summary of the invention

A composition according to the abovementioned first aspect of the present invention comprises the following active ingredients: beta glucans, at least one omega-3 fatty acid chosen among eicosapentaenoic acid (EPA) and docosahexaenoic acid (DHA) or triglycerides that contain it, vitamin E and folates.

Optionally, the composition may also comprise alpha-linolenic acid (ALA) or triglycerides that contain it.

In particular, the composition according to the present invention comprises, in weight percentages based on the total weight of the composition:

| | |
|---|---|
| Beta glucans | 70-85% |
| DHA and/or EPA | 12-25% |
| ALA | 0-10% |
| Vitamin E | 2-5% |
| Folates | 0.02-0.10% |

According to one embodiment of the present invention, the composition comprises, in weight percentages based on the total weight of the composition:

| | |
|---|---|
| Beta glucans | 75-85% |
| DHA and/or EPA | 14-25% |
| Vitamin E | 2-5% |
| Folates | 0.02-0.10% |

Vitamin E can be used in the form of d-α-tocopherol or of a mixture of the two d and 1 enantiomers of α-tocopherol, or of mixtures of other tocopherols (β, γ, ε, ζ, η) of plant origin, or of tocotrienols. The different forms of vitamin E can be of natural or synthetic origin.

The beta glucans can be suitably composed of beta glucans from barley, oats or their mixtures.

As source of the DHA and/or EPA fatty acids, fish oil can be used which contains such acids in the form of triglycerides.

Linseed oil can be used as a source of ALA or, alternatively, an adequate quantity of linseeds can be used.

It has been experimentally found that the composition according to the present invention, when incorporated in appropriate quantities in foods intended for daily consumption, reduces the risk of cardiovascular disease to an extent that is greater than that found for compositions of the prior art, presumably owing to a synergyistic action between the beta glucans and the other active ingredients, in particular the DHA and EPA fatty acids.

It is preferable that the food matrix in which the composition of the invention is incorporated is correctly balanced from the nutritional standpoint, so that the benefits attained by the intake of the active ingredients of the composition are not dispersed or reduced.

To such end, the present invention, in a further aspect thereof, relates to a bakery product containing, in weight percentages based on the total weight (on a dry weight basis), 3 - 22% lipids, 44-70% complex carbohydrates, 0-18% mono- and disaccharides and 0.8-8% of the aforementioned composition of the present invention.

In another aspect thereof, the present invention relates to a water-based drink containing the composition according to the present invention. Such drink contains 0.1-1.0%, preferably 0.2-0.7% of the above-described composition, at least one emulsifier and a fruit juice or extract. It can moreover contain mono- or disaccharides and/or sweeteners, such as for example sorbitol, xylitol, saccharine, aspartame and acesulfame.

As emulsifier for emulsifying the fish oil in the drink, a mixture of modified corn starch and soya lecithin may for example be used, previously thoroughly mixed into the fish oil itself.

It has experimentally been found that a regular intake of foods containing the compostion according to the present invention brings about a significant reduction in the plasma levels of triglycerides and homocysteine and it has been surprisingly found that such a reduction is not associated with an increase in LDL cholesterol levels, contrary to what occurs when pharmaceutical compositions based on DHA and/or EPA (e.g. fish oil) or food exclusively enriched with DHA and/or EPA are administered.

### Detailed description of the invention

The present invention will be further described with reference to four examples provided for illustrative and non-limiting purposes.

### EXAMPLE 1

### Bread

| | |
|---|---|
| "Type 0" wheat flour | 43.5 |
| Whole wheat flour | 5.0 |
| Olive oil | 3.0 |
| Dextrose monohydrate | 1.2 |
| Natural yeast | 2.0 |
| Vital, dry wheat gluten | 1.0 |
| Salt | 0.70 |
| Soya flour | 3.5 |
| Sunflower seeds | 3.0 |
| Roasted malt extract | 0.2 |
| Beta glucans from barley (75% titre) | 0.7 |
| Beta glucans from oats (22% titre) | 0.6 |
| Tocopherols (36% titre) | 0.08 |
| Fish oil with 27% DHA-EPA | 0.47 |
| Linseed oil with 50% ALA | 0.1 |
| Folic acid | 0.0004 |
| Vitamin B12 (0.1% titre) | 0.0003 |
| Vitamin B6 (100% titre) | 0.00005 |
| Water | 34.94925 |

The above-reported values are referred to a dough for the preparation of a bread of the present invention and are weight percentages based on the total weight of the dough.

The above-listed ingredients were mixed in an industrial kneading machine for the preparation of bread, until a uniform dough was obtained, which was then subjected to the standard dividing, resting, moulding and proving steps and was finally oven-baked at a temperature of 220°C for 26 minutes.

The bread thus obtained underwent evaluation by a trained sensory panel of 15, in comparison with a bread obtained from a dough in which the ingredients belonging to the composition according to the invention (i.e. beta glucans, tocopherols, fish oil, linseed oil, folic acid and vitamins B12 and B6) had been replaced with an equivalent amount of "type 0" wheat flour.

The panel found no significant differences between the two bread types with regard to the main characteristics (aroma, taste and texture).

### EXAMPLE 2

### Croissant-type bakery product

| | |
|---|---|
| "Type 0" wheat flour | 44.0 |
| Caster sugar | 5.9 |
| Natural baking yeast for bread-making | 0.686 |
| Vanillin | 0.1 |
| Whole eggs | 13.0 |
| Monoglycerides | 1.4 |
| Margarine | 10.5 |
| Glucose syrup | 5.5 |
| Salt | 0.4 |
| Beta glucans from barley (75% titre) | 1.3 |
| Beta glucans from oats (22% titre) | 0.76 |
| Tocopherols (36% titre) | 0.150 |
| Folic acid | 0.0003 |
| Vitamin B12 (0.1% titre) | 0.0007 |
| Vitamin B6 (100% titre) | 0.001 |
| Fish oil with 27% DHA-EPA | 0.8 |
| Linseed oil with 50% ALA | 0.2 |
| Water | 15.302 |

The above-reported values are referred to a dough for the preparation of a croissant of the present invention and are weight percentages based on the total weight of the dough.

The above-listed ingredients were mixed in a planetary mixer until a uniform dough was obtained, which was then subjected to the standard proving and resting steps and was finally oven-baked at a temperature of 190-200°C for 13-15 minutes.

### EXAMPLE 3

### Cake-type bakery product

| | |
|---|---|
| "Type 0" wheat flour | 26.0 |
| Caster sugar | 22.1 |
| Leavening agents (sodium bicarbonate, sodium acid pyrophosphate) | 0.6 |
| Lecithin | 0.3 |
| Flavourings | 0.789 |
| Whole eggs | 6.0 |
| Whole milk | 14.0 |
| Margarine | 12.2 |
| Glucose syrup | 5.5 |
| Salt | 0.3 |
| Beta glucans from barley (75% titre) | 1.3 |
| Beta glucans from oats (22% titre) | 0.76 |
| Tocopherols (36% titre) | 0.150 |
| Folic acid | 0.0003 |
| Vitamin B12 (0.1% titre) | 0.0007 |
| Vitamin B6 (100% titre) | 0.001 |
| Fish oil with 27% DHA-EPA | 0.8 |
| Linseed oil with 50% ALA | 0.2 |
| Water | 9.0 |

The above-reported values refer to a dough for the preparation of a small cake of the present invention and are weight percentages based on the total weight of the dough.

The above-listed ingredients were mixed in a planetary mixer until a uniform dough was obtained, which was then directly poured into a mold and oven-baked at a temperature of 190-200°C for 13-15 minutes.

### EXAMPLE 4

### Orange juice - based drink

**1 = the mixture consists of fish oil, modified corn starch and soya lecithin, with a 38% content of 27% DHA-EPA fish oil.**

| | |
|---|---|
| Concentrated orange juice | 14.28952 |
| Mixture of fish oil and emulsifiers¹ | 0.4 |
| Beta glucans from barley (75% titre) | 0.3 |
| Folic acid | 0.0001 |
| Vitamin E (50% titre) | 0.01 |
| Vitamin B12 (0.1% titre) | 0.00018 |
| Vitamin B6 (100% titre) | 0.0002 |
| Mineral water | 85.00 |

The above-reported values are weight percentages based on the total weight of the drink.

The bakery products described in the first three examples were used in a clinical study in patients suffering from alterations in their lipid metabolism. The results of that study are discussed herebelow.For the study, 16 subjects were selected of both sexes (49±14 years of age) having slight alterations in their lipid metabolism (plasma cholesterol of 218±30 mg/dl; plasma triglycerides of 168±76 mg/dl; HDL cholesterol of 41±13 mg/dl, M±DS) without a hypolipidemic drug therapy.

After a run-in period of about two weeks, during which time the subjects followed their usual diet, each subject was allocated, at random, either a diet comprising the products of examples 1-3 (4 portions/day) (T diet), or a diet containing bakery products having a composition analogous to that of the bakery products of examples 1-3 but lacking the active ingredients of the composition of the present invention (C diet).

Each subject followed the allocated diet for four weeks, and then followed the other diet regime for another four weeks. At the end of each treatment a test meal was administered having a composition similar to the current diet treatment (containing the bakery products with or without the active ingredients). Prior to the meal, and during the following 6 hours, blood samples were taken to determine cholesterol and triglycerides in the plasma and in the main lipoprotein fractions; furthermore, the fasting values for blood sugar, insulinaemia, C-reactive protein, and homocysteinaemia were determined. At the end of each diet, a test was also carried out for the evaluation of satiety (Visual Analogue Scale).

After the diet containing the products of examples 1-3 (T diet), a significant reduction in fasting plasma triglycerides was observed (138±66 v. 153±58 mg/dl, M±DS, p<0.05). LDL cholesterol levels were 121±22 and 122±18 mg/dl (ns) at the end of the T diet and of the C diet), respectively. Even postprandially both the total and the kilomicron triglycerides remained lower at the end of the T diet (p<0.05). Moreover, a significant reduction was observed in homocysteine (8±2 v. 10±3 µmol/L, M±DS, p<0.05) and in appetite (4±3 v. 6±3 after 6 hours, M±DS, p<0.05) with the T diet. The compliance to the diet regimes, which was evaluated by filling out a food diary, was excellent.

It can thus be concluded that a diet comprising the bakery products of examples 1-3, enriched with ω-3 fatty acids, folates, beta glucans and tocopherols decreases plasma levels of triglycerides (both in a fasting state and postprandially) and homocysteine. Moreover, this type of diet induces a greater satiety with possible benefits on the energy intake. As far as the LDL cholesterol levels are concerned, no significant differences are observed between the two diets, contrary to what has been observed in studies that have used fish oil in pharmaceutical preparations, where the levels of the LDL cholesterol often increased.

## Claims

1. Composition suitable for the addition to drinks and foods, the continued intake of which contributes towards a decrease in the risk of cardiovascular disease, comprising the following active ingredients: beta glucans, at least one omega-3 fatty acid chosen among eicosapentaenoic acid (EPA) and docosahexaenoic acid (DHA) or triglycerides that contain it, vitamin E and folates.

2. Composition according to claim 1, further comprising alpha-linolenic acid (ALA) or triglycerides that contain it.

3. Composition according to claim 1 or 2, comprising, in weight percentages based on the total weight of the composition:
| | |
|---|---|
| Beta glucans | 70-85% |
| DHA and/or EPA | 12-25% |
| ALA | 0-10% |
| Vitamin E | 2-5% |
| Folates | 0.02-0.10% |

4. Composition according to claim 1, comprising, in weight percentages based on the total weight of the composition:
| | |
|---|---|
| Beta glucans | 75-85% |
| DHA and/or EPA | 14-25% |
| Vitamin E | 2-5% |
| Folates | 0.02-0.10% |

5. Bakery product containing, in weight percentages based on the total weight (on a dry weight basis), 3 - 18% lipids, 45 - 70% complex carbohydrates, 0-16% mono- and disaccharides and 0.8 - 8% of the composition according to any one of the preceding claims.

6. Bakery product according to claim 5 containing, in weight percentages based on the total weight (on a dry weight basis), 0.8 - 6% of the composition according to any one of claims 1-4.

7. Water-based drink containing, in weight percentages based on the total weight of the drink, 0.1 - 1.0% of the composition according to any one of claims 1-4, at least one emulsifier and a fruit juice or extract.

8. Drink according to claim 7 containing, in weight percentages based on the total weight of the drink, 0.3 - 0.7% of the composition according to any one of claims 1-4.

9. Drink according to any one of the claims 7 and 8, further containing mono- and disaccharides and/or sweeteners.

10. Use of a composition comprising beta glucans, at least one omega-3 fatty acid, chosen among eicosapentaenoic acid (EPA) and docosahexaenoic acid (DHA) or triglycerides that contain it, vitamin E, folates and optionally linolenic acid or triglycerides that contain it in the preparation of foods and drinks useful in the reduction of the risk of cardiovascular disease.
